# EUROPEAN PATENT SPECIFICATION

(11) **EP 3 806 651 B1**
(45) Date of publication and mention of the grant of the patent: **08.01.2025**
(21) Application number: 19784838.5
(22) Date of filing: 11.04.2019
(51) Int. Cl.: A23K 10/18, C12N 1/20, A61K 35/742, A23L 29/00, A23L 33/135

(54) **A FEED INGREDIENT COMPRISING CLOSTRIDIUM TYROBUTYRICUM**
FUTTERMITTELZUTAT MIT CLOSTRIDIUM TYROBUTYRICUM
INGRÉDIENT ALIMENTAIRE COMPRENANT CLOSTRIDIUM TYROBUTYRICUM

(30) Priority: 13.04.2018 US 201862657309 P; 17.09.2018 US 201862731988 P
(43) Date of publication of application: 21.04.2021
(73) Proprietor: Superbrewed Food Inc., New Castle, DE 19720 (US)
(72) Inventor: TRACY, Bryan P., Wilmington, Delaware 19806 (US); JONES, Shawn William, Bear, Delaware 19701 (US); MARU, Biniam, Bear, DE 19701 (US); EYAL, Aharon M., 93629 Jerusalem (IL)
(74) Representative: Harrison IP Limited
(86) International application number: PCT/US2019/026996
(87) International publication number: WO 2019/200103

(56) References cited:
- WO-A1-2017/074566
- WO-A1-2018/055388
- WO-A1-2019/136236
- US-A1- 2016 068 919
- US-A1- 2016 338 380
- US-B2- 7 309 602
- US-B2- 8 501 463

## Description

### Background of the Invention

US4808417 teaches that in particular fish have a high nutritional requirement for protein in their feed. Examples of feed additives are provided, comprising *Clostridium butyricum, Clostridium acetobutyricum, Bacilli* or various *Lactobacilli* or *Bifidobacteria.* There is an on-going need for new feed ingredients of improved feed qualities, including higher crude protein content and higher content of essential amino acids.

### Summary of the Invention

According to an embodiment, provided is a feed ingredient comprising at least 20% by weight *Clostridium tyrobutyricum,* wherein said *Clostridium tyrobutyricum* comprises at least 70% of dry weight crude protein and optionally at least 7% of dry weight lysine and/or at least 1% of dry weight methionine. According to an embodiment, said *Clostridium tyrobutyricum* is genetically modified. According to an embodiment, said *Clostridium tyrobutyricum* is adaptively evolved.

According to an embodiment, said feed ingredient further comprises at least 20% by weight *Clostridium pasteurianum.* According to an embodiment, said feed ingredient further comprises at least 20% by weight an acetogenic bacteria. According to an embodiment, said feed ingredient further comprises at least 20% by weight *Butyribacterium methylotrophicum.* According to an embodiment, said feed ingredient further comprises at least 10% by weight *Clostridium pasteurianum* and at least 10% by weight *Butyribacterium methylotrophicum.*

According to an embodiment, said feed ingredient is characterized by water solubility greater than 25% by weight. According to an embodiment, said *Clostridium tyrobutyricum* is sporulated. According to an embodiment, said *Clostridium tyrobutyricum* is in clostridial form.

According to an embodiment, provided is an animal feed comprising said feed ingredient. According to an embodiment, said feed ingredient further comprises at least one of butyric acid and propionic acid. According to an embodiment, provided is an aqua feed comprising said feed ingredient.

According to an embodiment, provided is a probiotic preparation comprising said feed ingredient. According to an embodiment, provided is a poultry probiotic preparation comprising said feed ingredient. According to an embodiment, provided is a swine probiotic preparation comprising said feed ingredient. According to an embodiment, provided is a companion animal probiotic preparation comprising said feed ingredient. According to an embodiment, provided is a human probiotic preparation comprising said feed ingredient. According to an embodiment, said probiotic preparation further comprising at least one of *Enterococcus faecium, Bacillus subtilis, Bacillus amyloliquefaciens, Lactobacillus acidophilus, Lactobacillus plantarum, Pediococcus acidilactici and Clostridium butyricum.*

According to an embodiment, provided is a method for producing said feed ingredient, comprising culturing in a fermentation medium *Clostridium tyrobutyricum,* whereby *Clostridium tyrobutyricum* cell mass is formed. According to an embodiment, said fermentation medium is kept at a pH greater than 5.25.

According to an embodiment, said method further comprises co-culturing *Clostridium pasteurianum,* whereby said cell mass further comprises *Clostridium pasteurianum.* According to an embodiment, said method further comprises co-culturing an acetogenic bacteria, whereby said cell mass further comprises an acetogenic bacteria. According to an embodiment, said method further comprises co-culturing *Butyribacterium methylotrophicum,* whereby said cell mass further comprises *Butyribacterium methylotrophicum.* According to an embodiment, said method further comprises co-culturing *Clostridium pasteurianum* and *Butyribacterium methylotrophicum,* whereby said cell mass further comprises *Clostridium pasteurianum and Butyribacterium methylotrophicum.* According to an embodiment, said method further comprises mechanical separation of formed cell mass and at least one of washing, drying, pulverizing and pelletizing.

Provided is a method for maintaining the good health of an animal comprising feeding said animal an animal feed comprising said feed ingredient. Said animal is selected from the group consisting of larvae, fry, juvenile, and adult of crustacean, finfish and shellfish, poultry, swine, companion animals, other ruminants and humans Said maintaining good health comprises at least one of reduced susceptibility to enteric pathogens, reduction in bowel inflammation, reduced mortality, improved feed conversion and improved weight gain.

Provided is a method for maintaining the good health of an animal comprising feeding said animal a probiotic preparation comprising said feed ingredient. Said animal is selected from the group consisting of larvae, fry, juvenile, and adult of crustacean, finfish and shellfish, poultry, swine, companion animals, other ruminants and humans. Said maintaining good health comprises at least one of reduced susceptibility to enteric pathogens, reduction in bowel inflammation, reduced mortality, improved feed conversion and improved weight gain.

### Detailed Description of the Invention

The particulars shown herein are by way of example and for purposes of illustrative discussion of the various embodiments of the present invention only and are presented in the cause of providing what is believed to be the most useful and readily understood description of the principles and conceptual aspects of the invention. In this regard, no attempt is made to show details of the invention in more detail than is necessary for a fundamental understanding of the invention, the description making apparent to those skilled in the art how the several forms of the invention may be embodied in practice.

The present invention will now be described by reference to more detailed embodiments. This invention may, however, be embodied in different forms and should not be construed as limited to the embodiments set forth herein. Rather, these embodiments are provided so that this disclosure will be thorough and complete, and will fully convey the scope of the invention to those skilled in the art.

Unless otherwise defined, all technical and scientific terms used herein have the same meaning as commonly understood by one of ordinary skill in the art to which this invention belongs. The terminology used in the description of the invention herein is for describing particular embodiments only and is not intended to be limiting of the invention. As used in the description of the invention and the appended claims, the singular forms "a," "an," and "the" are intended to include the plural forms as well, unless the context clearly indicates otherwise.

Unless otherwise indicated, all numbers expressing quantities of ingredients, reaction conditions, and so forth used in the specification and claims are to be understood as being modified in all instances by the term "about." Accordingly, unless indicated to the contrary, the numerical parameters set forth in the following specification and attached claims are approximations that may vary depending upon the desired properties sought to be obtained by the present invention. At the very least, and not as an attempt to limit the application of the doctrine of equivalents to the scope of the claims, each numerical parameter should be construed in light of the number of significant digits and ordinary rounding approaches.

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements. Every numerical range given throughout this specification will include every narrower numerical range that falls within such broader numerical range, as if such narrower numerical ranges were all expressly written herein.

Additional advantages of the invention will be set forth in part in the description, which follows, and in part will be obvious from the description, or may be learned by practice of the invention. It is to be understood that both the foregoing general description and the following detailed description are exemplary and explanatory only and are not restrictive of the invention, as claimed.

According to an embodiment, provided is a feed ingredient comprising at least 20% by weight (20%wt) *Clostridium tyrobutyricum,* wherein said *Clostridium tyrobutyricum* comprises at least 70% of dry weight crude protein and optionally at least 7% of dry weight lysine and/or at least 1% of dry weight methionine. According to an embodiment, provided is a feed ingredient comprises at least 30%wt, at least 40%wt, at least 50%wt, at least 60%wt, at least 70%wt, at least 80%wt, at least 90%wt or at least 95%wt *Clostridium tyrobutyricum.* According to an embodiment, said *Clostridium tyrobutyricum* comprises at least 75% of dry weight crude protein, at least 80%, at least 85% or at least 90%. According to an embodiment, said *Clostridium tyrobutyricum* comprises at least 7% of dry weight lysine, at least 7.5%, at least 8%, at least 8.5% or at least 9%. According to an embodiment, said *Clostridium tyrobutyricum* comprises at least 1% of dry weight methionine, at least 1.5%, at least 2%, or at least 2.5%.

According to an embodiment, said feed ingredient further comprises at least 20%wt *Clostridium pasteurianum,* at least 30%wt, at least 40%wt, at least 50%wt, at least 60%wt or at least 70%wt. According to an embodiment, said feed ingredient comprises at least 1%wt glycogen, at least 5%wt, at least 10%wt, at least 15%wt, at least 20%wt, at least 25%wt or at least 30%wt. According to an embodiment, said feed ingredient further comprises at least 20%wt *Butyribacterium methylotrophicum,* at least 30%wt, at least 40%wt or at least 50%wt.

According to an embodiment, said feed ingredient further comprises at least 20%wt of acetogenic bacteria, at least 30%wt, at least 40%wt, at least 50%wt, at least 60%wt or at least 70%wt.

According to an embodiment, said feed ingredient further comprises at least 20%wt *Butyribacterium methylotrophicum* at least 30%wt, at least 40%wt, at least 50%wt, at least 60%wt or at least 70%wt.

According to an embodiment, said feed ingredient further comprises at least 10%wt *Clostridium pasteurianum,* at least 20%wt or at least 30%wt and at least 10% by weight *Butyribacterium methylotrophicum,* at least 20%wt or at least 30%wt.

According to an embodiment, said animal feed is characterized by water solubility greater than 25%wt, greater than 40%wt, greater than 60%wt, greater than 80%wt, or greater than 80%wt.

According to an embodiment, said *Clostridium tyrobutyricum* is sporulated. According to an embodiment, said *Clostridium tyrobutyricum* is in clostridial form.

According to an embodiment, said *Clostridium tyrobutyricum* is genetically modified. According to an embodiment, said *Clostridium tyrobutyricum* is genetically modified by removing or inserting genetic material out of or into the chromosome. According to an embodiment, said genetic modification is not plasmid-based. According to an embodiment, said *Clostridium tyrobutyricum* is genetically modified to produce propionic acid. According to an embodiment, said *Clostridium tyrobutyricum* is genetically modified to reduce the production of graulose. According to an embodiment, said *Clostridium tyrobutyricum* is adaptively evolved. According to an embodiment, said *Clostridium tyrobutyricum* is adaptively evolved to have a higher growth rate. According to an embodiment, said *Clostridium tyrobutyricum* is adaptively evolved to have an improved substrate utilization rate. According to an embodiment, said *Clostridium tyrobutyricum* is adaptively evolved to have a higher crude protein content.

According to an embodiment, said clostridial form is characterized by the formation of granulose. According to an embodiment, said granulose comprises amylopectin and optionally also amylose.

According to an embodiment, provided is an animal feed comprising said feed ingredient. According to an embodiment, said feed ingredient forms at least 2%wt of said animal feed, at least 4%wt, at least 6%wt, at least 8%wt or at least 10%wt. According to an embodiment, said animal feed further comprises at least one of butyric acid and propionic acid. According to an embodiment, said animal feed further comprises amylopectin and amylose with amylopectin to amylose weight/weight ratio of about 1 to 4.

According to an embodiment, provided is an aqua feed comprising said feed ingredient. According to an embodiment, said feed ingredient forms at least 2%wt of said aqua feed, at least 4%wt, at least 6%wt, at least 8%wt or at least 10%wt.

According to an embodiment, provided is a probiotic preparation comprising said feed ingredient. According to an embodiment, said feed ingredient forms at least 20%wt of said probiotic preparation, at least 40%wt, at least 60%wt, at least 80%wt or at least 90%wt. According to an embodiment, said probiotic preparation further comprising at least one of *Enterococcus faecium, Bacillus subtilis, Bacillus amyloliquefaciens, Lactobacillus acidophilus, Lactobacillus plantarum, Pediococcus acidilactici and Clostridium butyricum.*

According to an embodiment, provided is a poultry probiotic preparation comprising said feed ingredient. According to an embodiment, said feed ingredient forms at least 20%wt of said poultry probiotic preparation, at least 40%wt, at least 60%wt, at least 80%wt or at least 90%wt.

According to an embodiment, provided is a human probiotic preparation comprising said feed ingredient. According to an embodiment, said feed ingredient forms at least 20%wt of said human probiotic preparation, at least 40%wt, at least 60%wt, at least 80%wt or at least 90%wt.

According to an embodiment, provided is a method for producing said feed ingredient, comprising culturing in a fermentation medium *Clostridium tyrobutyricum,* whereby *fermentation broth comprising Clostridium tyrobutyricum* cell mass is formed. According to an embodiment, said fermentation broth further comprises at least one of acetic acid and butyric acid. According to an embodiment, said fermentation medium is kept at a pH greater than 5.25, greater than 5.30, greater than 5.35, greater than 5.40, greater than 5.45, or greater than 5.50.

According to an embodiment, said method comprises co-culturing *Clostridium pasteurianum,* whereby said cell mass further comprises *Clostridium pasteurianum.* According to an embodiment, said method comprises co-culturing an acetogenic bacteria, whereby said cell mass further comprises an acetogenic bacteria. According to an embodiment, said method comprises co-culturing *Butyribacterium methylotrophicum,* whereby said cell mass further comprises *Butyribacterium methylotrophicum.* According to an embodiment, said method comprises co-culturing *Clostridium pasteurianum* and *Butyribacterium methylotrophicum,* whereby said cell mass further comprises *Clostridium pasteurianum and Butyribacterium methylotrophicum.* According to an embodiment, said fermentation medium comprises a first feedstock comprising carbohydrates, glycerol, methanol, or combinations thereof. According to an embodiment, said fermentation medium comprises a second feedstock comprising CO, CO₂, carbonate, bicarbonate, H₂, glycerol, methanol, formate, urea or mixtures thereof. According to an embodiment, said first feedstock, said second feedstock or both are metabolized during said culturing.

According to an embodiment, said method comprises mechanical separation of formed cell mass and at least one of washing, drying pulverizing, extrusion and pelletizing. According to an embodiment, said method comprises processing the cell mass to have a water solubility greater than 25%, greater than 50%, greater than 60%, greater than 70%, greater than 80%, or greater than 90%.

Provided is a method for maintaining the good health of an animal comprising feeding said animal an animal feed comprising said feed ingredient. Said animal is selected from the group consisting of larvae, fry, juvenile, and adult of crustacean, finfish and shellfish, poultry, swine, companion animals, such as dogs and cats, other ruminants and humans. Said maintaining good health comprises at least one of reduced susceptibility to enteric pathogens, reduction in bowel inflammation, reduced mortality, improved feed conversion, and improved weight gain.

Provided is a method for maintaining the good health of an animal comprising feeding said animal a probiotic preparation comprising said feed ingredient. Said animal is selected from the group consisting of larvae, fry, juvenile, and adult of crustacean, finfish and shellfish, poultry, swine, companion animals, other ruminants and humans. Said maintaining good health comprises at least one of reduced susceptibility to enteric pathogens, reduction in bowel inflammation, consumption of lactic acid, reduced mortality, improved feed conversion, and improved weight gain.

### Examples

### Example 1. Clostridium tyrubutyricum fermentation.

Clostridium tyrobutyricum was grown on a hydrolyzed corn starch medium with 10 mL/L trace elements solution (2.0 g/L nitrilotriacetic acid, 1.0 g/L MnSO4·H2O, 0.8 g/L Fe(SO4)2(NH4)2·6H2O, 0.2 g/L CoCl2·6H2O, 0.2 mg/L ZnSO4·7H2O, 20.0 mg/L CuCl2·2H2O, 20.0 mg/L NiCL2·6H2O, 20.0 mg/L Na2MoO4·2H2O, 20.0 mg/L Na2SeO4, and 20.0 mg/L Na2WO4) and 10 mL/L Wolfe's vitamins (2.0 mg/L biotin, 2.0 mg/L folic acid, 10.0 mg/L pyridoxine hydrochloride, 5.0 mg/L thiamine-HCl, 5.0 mg/L riboflavin, 5.0 mg/L nicotinic acid, 5.0 mg/L calcium D-(+)-pantothenate, 0.1 mg/L vitamin B12, 5.0 mg/L p-aminobenzoic acid, and 5.0 mg/L thioctic acid). The estimated glucose concentration was 65 g/L. The cells were grown under anaerobic conditions (low CO2 sparge) at 37°C. The pH was bottom controlled at 5.5 with 8M NH4OH. Table 1 shows the increase in optical density (OD) over time along with butyric acid.

**Table 1. Clostridium tyrobutyricum fermentation.**

| | | Concentration (g/L) | | | |
|---|---|---|---|---|---|
| Fermentation time (h) | OD* | Lactic acid | Acetic acid | Butyric acid | Ethanol |
| 0 | 0.44 | 0.54 | 0.18 | 0.21 | 0.09 |
| 6 | 2.53 | 0.29 | 0.48 | 1.08 | 0.16 |
| 12 | 11.50 | 0.88 | 1.46 | 4.72 | 0.29 |
| 18 | 36.30 | 0.10 | 2.47 | 10.09 | 0.41 |

| | | | | | |
|---|---|---|---|---|---|
| * OD measured at 600 nm | | | | | |

At 18 hours, the cells were harvested, washed three times in water, and dried with a dual drum dryer. The resulting dried cell mass had the characteristics listed in Table 2.

**Table 2. Characteristics of Clostridium tyrobutyricum cell mass.**

| Characteristic | As is % | Dried % |
|---|---|---|
| Moisture | 4.7 | - |
| Crude protein | 79.9 | 83.9 |
| Ash | 7.85 | 8.24 |
| Lysine | - | 8.33 |
| Methionine | - | 1.42 |

### Example 2. Co-culture fermentation.

A co-culture of *Clostridium tyrobutyricum* and *Butyribacterium methylotrophicum* was prepared and grown on hydrolyzed corn starch medium. The seed culture was generated by mixing equal volumes of a pure culture of C. *tyrobutyricum* and *B. methylotrophicum.* The estimated glucose concentration was 100 g/L. The cells were grown under anaerobic conditions (low CO₂ sparge) at 37°C. The pH was bottom controlled at 5.5 with 8M NH₄OH. Table 3 shows the increase in optical density (OD) over time along with butyric acid.

**Table 3. Co-culture fermentation.**

| | | Concentration (g/L) | | | |
|---|---|---|---|---|---|
| Fermentation time (h) | OD* | Lactic acid | Acetic acid | Butyric acid | Ethanol |
| 0 | 0.56 | 0.69 | 0.70 | 0.23 | 0.65 |
| 16 | 11.20 | 0.00 | 1.30 | 6.08 | 0.20 |
| 24 | 17.60 | 0.00 | 2.78 | 13.61 | 1.05 |
| 42 | 22.20 | 0.00 | 2.99 | 26.86 | 1.49 |

| | | | | | |
|---|---|---|---|---|---|
| * OD measured at 600 nm | | | | | |

At 42 hours, the cells were harvested and washed three times in water. The resulting wet cell mass was analyzed and had the characteristics listed in Table 4.

**Table 4. Characteristics of Clostridium tyrobutyricum cell mass.**

| Characteristic | As is % | Dried % |
|---|---|---|
| Moisture | 69.1 | - |
| Crude protein | 24.5 | 79.3 |
| Ash | 1.8 | 5.8 |

### Examples 3-28

Feed ingredient cell-mass compositions

| Example | *Clostridium tyrobutyricum* (%wt) | *Butyribacterium methylotrophicum* (%wt) | *Clostridium pasteurianum* (%wt) |
|---|---|---|---|
| 3 | 95 | <5 | <5 |
| 4 | 75 | 20 | <5 |
| 5 | 65 | 30 | <5 |
| 6 | 55 | 40 | <5 |
| 7 | 45 | 50 | <5 |
| 8 | 75 | <5 | 20 |
| 9 | 65 | <5 | 30 |
| 10 | 55 | <5 | 40 |
| 11 | 45 | <5 | 50 |
| 12 | 75 | 10 | 10 |
| 13 | 70 | 15 | 10 |
| 14 | 70 | 10 | 15 |
| 15 | 65 | 15 | 15 |
| 16 | 60 | 20 | 15 |
| 17 | 60 | 15 | 20 |
| 18 | 55 | 20 | 20 |
| 19 | 55 | 25 | 15 |
| 20 | 55 | 15 | 25 |
| 21 | 50 | 25 | 20 |
| 22 | 50 | 20 | 25 |
| 23 | 45 | 25 | 20 |
| 24 | 45 | 20 | 25 |
| 25 | 45 | 30 | 20 |
| 26 | 45 | 20 | 30 |
| 27 | 40 | 35 | 20 |
| 28 | 40 | 20 | 35 |

### Example 29. Early survival of whiteleg shrimp (Litopenaeus vannamei)

Four 140-liter tanks with 100 whiteleg shrimp (Litopenaeus vannamei) at growing stage PL10 (2 to 3 gr.) growth in a recirculating aquaculture saltwater (25 ppt) system, water maintained at ~27°C and >80% saturation of dissolved oxygen. The shrimps were feed with a diet including *Clostridium tyrobutyricum* in accordance to claim 1. the tanks diets were: tank 1 - control, tank 2- *5% Clostridium tyrobutyricum,* tank 3- 10% *Clostridium tyrobutyricum,* tank 4- 15% *Clostridium tyrobutyricum.*
the aim of the experiment was to check the influence of Clostridium tyrobutyricum feed on early mortality syndrome (EMS) in diets of whiteleg shrimp

**Table 5. survival of whiteleg shrimp.**

| day | Tank 1 - control | Tank 2 5% diet | Tank 3 10% diet | Tank 4 15% diet |
|---|---|---|---|---|
| 0 | 100 | 100 | 100 | 100 |
| 7 | 44 | 66 | 100 | 97 |
| 14 | 41 | 61 | 96 | 96 |

## Claims

1. A feed ingredient comprising at least 20% by weight *Clostridium tyrobutyricum,* wherein said *Clostridium tyrobutyricum* comprises at least 70% of dry weight crude protein and optionally at least 7% of dry weight lysine and/or at least 1% of dry weight methionine.

2. The feed ingredient of Claim 1, further comprising at least 20% by weights *Butyribacterium methylotrophicum.*

3. The feed ingredient of Claim 1, wherein said *Clostridium tyrobutyricum* is sporulated.

4. The feed ingredient of Claim 1, wherein said *Clostridium tyrobutyricum* is in clostridial form.

5. The feed ingredient of Claim 1, wherein said *Clostridium tyrobutyricum* is genetically modified, optionally by removing or inserting genetic material out of or into the chromosome.

6. An animal feed comprising the feed ingredient according to Claim 1.

7. The animal feed of Claim 6, further comprising at least one of butyric acid and propionic acid.

8. A probiotic preparation comprising the feed ingredient according to Claim 1.

9. The probiotic preparation of Claim 8, further comprising at least one of *Enterococcus faecium, Bacillus subtilis, Bacillus amyloliquefaciens, Lactobacillus acidophilus, Lactobacillus plantarum, Pediococcus acidilactici and Clostridium butyricum.*

10. A method for producing a feed ingredient according to Claim 1, comprising culturing in a fermentation medium *Clostridium tyrobutyricum,* whereby *Clostridium tyrobutyricum* cell mass is formed.

11. The method of Claim 10, wherein said fermentation medium is kept at a pH greater than 5.25, the method optionally further comprising mechanical separation of formed cell mass and at least one of washing, drying, pulverizing and pelletizing.

12. The animal feed of Claim 6 or the probiotic preparation of Claim 8, for use in maintaining the good health of an animal.

13. The animal feed for use or the probiotic preparation for use according to Claim 12, wherein said animal is selected from the group consisting of finfish and shellfish, larvae, fry, juvenile, and adult fish, poultry, swine, companion animals, other ruminants and humans.

14. The animal feed for use or the probiotic preparation for use according to claim 12, wherein said maintaining good health comprises at least one of reduced susceptibility to enteric pathogens, reduction in bowel inflammation, reduced mortality, improved feed conversion and improved weight gain.

## Patentansprüche

1. Futterbestandteil, der mindestens 20 % Massenanteil *Clostridium tyrobutyricum* umfasst, wobei das *Clostridium tyrobutyricum* mindestens 70 % Rohprotein (Trockenmasse) und optional mindestens 7 % Lysin (Trockenmasse) und/oder mindestens 1 % Methionin (Trockenmasse) umfasst.

2. Futterbestandteil nach Anspruch 1, der ferner mindestens 20 % Massenanteil *Butyribacterium methylotrophicum* umfasst.

3. Futterbestandteil nach Anspruch 1, wobei das *Clostridium tyrobutyricum* sporuliert ist.

4. Futterbestandteil nach Anspruch 1, wobei das *Clostridium tyrobutyricum* in Clostridienform vorliegt.

5. Futterbestandteil nach Anspruch 1, wobei das *Clostridium tyrobutyricum* genetisch verändert ist, optional durch Entfernen oder Einfügen von genetischem Material aus dem oder in das Chromosom.

6. Tierfutter, das den Futterbestandteil gemäß Anspruch 1 umfasst.

7. Tierfutter nach Anspruch 6, das zusätzlich mindestens eine von Buttersäure und Propionsäure umfasst.

8. Probiotisches Präparat, das den Futterbestandteil gemäß Anspruch 1 umfasst.

9. Probiotisches Präparat nach Anspruch 8, das ferner mindestens einen von *Enterococcus faecium, Bacillus subtilis, Bacillus amyloliquefaciens, Lactobacillus acidophilus, Lactobacillus plantarum, Pediococcus acidilactici und Clostridium butyricum* umfasst.

10. Verfahren zur Herstellung eines Futterbestandteils nach Anspruch 1, das das Kultivieren von *Clostridium tyrobutyricum* in einem Fermentationsmedium umfasst, wobei *Clostridium tyrobutyricum-Zellmasse* gebildet wird.

11. Verfahren nach Anspruch 10, wobei das Fermentationsmedium auf einem pH-Wert über 5,25 gehalten wird, wobei das Verfahren optional ferner eine mechanische Trennung der gebildeten Zellmasse und mindestens eines von Waschen, Trocknen, Pulverisieren und Pelletieren umfasst.

12. Tierfutter nach Anspruch 6 oder probiotisches Präparat nach Anspruch 8 zur Verwendung zur Erhaltung der Gesundheit eines Tieres.

13. Tierfutter zur Verwendung oder probiotisches Präparat zur Verwendung gemäß Anspruch 12, wobei das Tier ausgewählt ist aus der Gruppe bestehend aus Flossenfischen und Schalentieren, Larven, Brutfischen, Jung- und erwachsenen Fischen, Geflügel, Schweinen, Haustieren, anderen Wiederkäuern und Menschen.

14. Tierfutter zur Verwendung oder probiotisches Präparat zur Verwendung gemäß Anspruch 12, wobei die Erhaltung einer guten Gesundheit mindestens eines von verringerter Anfälligkeit für Darmpathogene, Verringerung von Darmentzündungen, verringerter Sterblichkeit, verbesserter Futterverwertung und verbesserter Gewichtszunahme umfasst.

## Revendications

1. Ingrédient alimentaire comprenant au moins 20 % en poids de *Clostridium tyrobutyricum,* ledit *Clostridium tyrobutyricum* comprenant au moins 70 % en poids sec de protéine brute et éventuellement au moins 7 % en poids sec de lysine et/ou au moins 1 % en poids sec de méthionine.

2. Ingredient alimentaire selon la revendication 1, comprenant en outre au moins 20 % en poids de *Butyribacterium methylotrophicum.*

3. Ingrédient alimentaire selon la revendication 1, dans lequel ledit *Clostridium tyrobutyricum* est sporulé.

4. Ingrédient alimentaire selon la revendication 1, dans lequel ledit *Clostridium tyrobutyricum* est sous forme clostridiale.

5. Ingrédient alimentaire selon la revendication 1, dans lequel ledit *Clostridium tyrobutyricum* est génétiquement modifié, éventuellement par enlèvement de matériel génétique du chromosome ou insertion dudit matériel dans celui-ci.

6. Aliment pour animal comprenant l'ingrédient alimentaire selon la revendication 1.

7. Aliment pour animal selon la revendication 6, comprenant en outre au moins l'un de l'acide butyrique et de l'acide propionique.

8. Préparation probiotique comprenant l'ingrédient alimentaire selon la revendication 1.

9. Préparation probiotique selon la revendication 8, comprenant en outre au moins l'un de *Enterococcus faecium, Bacillus subtilis, Bacillus amyloliquefaciens, Lactobacillus acidophilus, Lactobacillus plantarum, Pediococcus acidilactici* et *Clostridium butyricum.*

10. Procédé de production d'un ingrédient alimentaire selon la revendication 1, comprenant la mise en culture de *Clostridium tyrobutyricum* dans un milieu de fermentation, ce qui a pour effet de former une masse de cellules de *Clostridium tyrobutyricum.*

11. Procédé selon la revendication 10, dans lequel ledit milieu de fermentation est maintenu à un pH supérieur à 5,25, le procédé comprenant en outre éventuellement la séparation mécanique de la masse de cellules formée et au moins l'une des opérations de lavage, séchage, pulvérisation et agglomération.

12. Aliment pour animal selon la revendication 6, ou préparation probiotique selon la revendication 8, destiné(e) à être utilisé(e) pour le maintien de la bonne santé d'un animal.

13. Aliment pour animal ou préparation probiotique destiné(e) à être utilisé(e) selon la revendication 12, ledit animal étant sélectionné dans le groupe constitué de : poissons à nageoires et mollusques et crustacés, larves, alevins, juvéniles et poissons adultes, volaille, cochons, animaux de compagnie, autres ruminants et humains.

14. Aliment pour animal ou préparation probiotique destiné(e) à être utilisé(e) selon la revendication 12, ledit maintien de la bonne santé comprenant au moins l'une de : réduction de la vulnérabilité aux pathogènes entériques, réduction de l'inflammation intestinale, réduction de la mortalité, amélioration de la valorisation des aliments et amélioration du gain de poids.
